# DEMANDE DE BREVET EUROPEEN

(11) **EP 0 846 444 A1**
(43) Date de publication de la demande: **10.06.1998**
(21) Numéro de dépôt: 97402910.0
(22) Date de dépôt: 02.12.1997
(51) Int. Cl.: A61B 17/70

(54) **Dispositif de correction de déviation rachidienne**

(30) Priorité: 03.12.1996 FR 9614912
(71) Demandeur: BIOMAT, 91892 Saclay (FR)
(72) Inventeur: Lahille, Michel, 91430 Vauhallan (FR); Lemaire, Jean-Philippe, 21121 Fontaine Les Dijon (FR); Ben-Mokhtar, Mourad, 75006 Paris (FR)
(74) Mandataire: Martinet & Lapoux

(57) **Abrégé**

Un dispositif de correction rachidienne comprend une tige (1), un élément du type vis ou crochet pédiculaire ou transversaire (5) ayant un tronçon fileté (51) s'étendant à partir d'une tête (53) et muni d'un écrou (27), et une attache (4) ayant un orifice traversé par le tronçon fileté pour lier l'élément à la tige. L'attache (4) comprend deux surfaces d'appui (411, 421) qui sont opposées sensiblement transversalement à la tige (1) et décalées le long de la tige d'au moins la plus petite dimension de la section transversale de la tige (1). L'attache est introduite transversalement à la tige après que celle-ci soit déjà liée à d'autres vis implantées, de manière à mobiliser toute vertèbre en la reliant par une vis ou un crochet à l'attache.

## Description

La présente invention concerne la consolidation du rachis et plus particulièrement la correction de déviation(s) du rachis au moyen d'un dispositif d'ostéosynthèse.

Les dispositifs d'ostéosynthèse de correction de déviation rachidienne comprennent pour la plupart une tige de consolidation reliée à des éléments vertébraux de fixation qui sont de deux types principaux. Selon un premier type, l'élément vertébral est une "vis" ayant un tronçon fileté autotaraudeur dans un pédicule vertébral et une portion de tête à relier à la tige. Selon un second type, l'élément vertébral est un "crochet" pédiculaire ayant une portion à lame pouvant être accrochée à un pédicule vertébral, au niveau d'anfractuosités ou d'apophyses, et une portion de tête à relier à la tige.

Afin d'homogénéiser l'instrumentation et réduire le coût de fabrication des diverses pièces du dispositif, les portions de tête des vis et crochets pédiculaires sont bien souvent identiques ou similaires pour un type de dispositif donné. Par exemple, la portion de tête de vis ou crochet et la tige de consolidation sont reliées :
- au moyen d'un écrou vissé autour de deux mâchoires filetées de la portion de tête et séparées par une fente dans laquelle la tige est serrée,
- ou au moyen d'une pièce de verrouillage en U encliquetée sur la tige et logeable dans une fente de la portion de tête par écartement de flancs de celle-ci,
- ou par enfilage de la tige dans un trou de la portion de tête et vissage d'une petite vis pour immobiliser la portion de tête,
- ou bien encore au moyen d'une attache de liaison ayant un premier alésage traversé par la tige, un second alésage traversé par un tronçon fileté de la portion de tête, et une fente débouchant axialement dans le premier alésage et s'étendant radialement à travers le second alésage afin que le vissage d'un écrou sur le tronçon fileté rétrécit la fente et pince la tige.

Une ou deux tiges sensiblement parallèles reliées entr'elles par des barres de stabilisation transversale sont positionnées le long du rachis suivant la courbure de celui-ci à corriger. En général, au moins trois points d'ancrage sont préalablement définis au moyen de vis et/ou crochets implantés dans les corps vertébraux au moins aux extrémités et le cas échéant au voisinage du sommet de la courbure scoliotique et/ou cyphotique ou lordosique à corriger. Lorsque la tige est longue, ou celle-ci doit corriger une double courbure à la fois cyphotique au niveau des vertèbres thoraciques et lordosique au niveau des vertèbres lombaires, la tige est soumise à des translations, rotations, flexions et déflexions de manière à restaurer la courbure souhaitée. La tige doit être ainsi mobile au moins par glissement et/ou rotation dans tous les moyens de liaison supportés par les portions de tête des éléments vertébraux implantés. En outre, il n'est pas possible de sélectionner précisément les vertèbres qui sont à mobiliser entre les extrémités de la courbure en fonction de la correction souhaitée.

L'objectif principal de l'invention est de fournir un dispositif de correction rachidienne qui permet aisément d'ajouter des éléments vertébraux additionnels à implanter après que la tige soit déjà introduite dans des moyens de liaison d'éléments vertébraux déjà implantés aux extrémités d'une courbure rachidienne à corriger. Les éléments vertébraux additionnels sont implantés au fur et à mesure et de manière séparée en fonction de la mobilisation de vertèbres prédéterminées intermédiaires entre les extrémités de la courbure.

A cette fin, un dispositif de correction rachidienne comprend une tige, un élément ayant un tronçon fileté s'étendant à partir d'une tête et muni d'un écrou, et une attache ayant un orifice traversé par le tronçon fileté pour lier ledit élément à la tige, et est caractérisé en ce que l'attache comprend deux surfaces d'appui qui sont opposées sensiblement transversalement à la tige et décalées le long de la tige d'au moins la plus petite dimension de la section transversale de la tige.

Les deux surfaces d'appui ont de préférence des sections transversales similaires à des demi-sections transversales opposées de la tige afin d'augmenter l'adhérence sur la tige et l'effet de levier bloquant l'attache sur la tige. En pratique, les surfaces d'appui sont celles de deux crochets saillant d'une queue de l'attache dans laquelle l'orifice traversé par le tronçon fileté est ménagé.

Selon une caractéristique, le dispositif peut comprendre un moyen traversé par le tronçon fileté et disposé entre la tête d'élément et l'écrou pour amortir des déplacements relatifs entre la tige et ledit élément. Le moyen pour amortir comprend une rondelle en matériau amortissant qui est de préférence en contact avec ou liée à l'une ou deux rondelles métalliques.

Selon une autre caractéristique, le dispositif peut comprendre une rondelle à rotule traversée par le tronçon fileté, disposée sous-jacente à l'orifice de l'attache, entre celles-ci et la tête d'élément. L'orifice est alors une lumière oblongue ayant au moins partiellement un profil transversal de contour semi-circulaire coopérant avec une extrémité semi-sphérique de la rondelle à rotule. La longueur de la lumière du dispositif est de préférence supérieure au diamètre de l'extrémité semi-sphérique de la rondelle à rotule et la largeur de la lumière est inférieure au diamètre de l'extrémité semi-sphérique de la rondelle à rotule afin d'autoriser un débattement du tronçon fileté de l'élément perpendiculairement à la tige.

Pour réduire le nombre de pièces, la rondelle à rotule et une rondelle métallique du moyen pour amortir peuvent être monolithiques, et la rondelle en matériau amortissant peut être collée à l'une des ou aux deux rondelles métalliques.

Selon une autre caractéristique, le dispositif peut comprendre un moyen traversé par le tronçon fileté et disposé entre ladite attache et l'écrou pour fournir une surface d'appui de l'écrou perpendiculaire au tronçon fileté, indépendamment de l'angle entre le tronçon fileté et l'attache afin que l'écrou applique une force de serrage toujours axiale et n'ait pas son filetage endommagé. Le moyen pour fournir une surface d'appui de l'écrou peut présenter une surface crantée pour être appliquée contre une face crantée de l'attache de manière à augmenter l'adhérence entre ledit moyen et l'attache. En pratique, le moyen pour fournir une surface d'appui de l'écrou comprend deux rondelles en coin. Les deux rondelles en coin sont solidaires avec jeu en direction axiale et sont libres en rotation axiale l'une par rapport à l'autre.

Divers éléments vertébraux peuvent être reliés à la tige par une attache selon l'invention. Par exemple, l'élément peut être du type vis et comporter une partie au moins partiellement filetée et séparée dudit tronçon fileté par la tête, ou peut être du type crochet.

Une partie de crochet d'élément peut être en forme de dièdre sensiblement droit séparée du tronçon fileté de l'élément par la tête. La partie de crochet en forme de dièdre peut comporter un côté libre en forme de lame terminée par deux dents pour enfourcher une pédicule.

Selon une autre variante, une partie de crochet d'élément peut être en forme de dièdre sensiblement droit dont l'angle plan est incliné par rapport au tronçon fileté, par exemple de 45°, et qui est séparée dudit tronçon fileté par la tête.

Selon une autre caractéristique, le dispositif peut comprendre une seconde attache ayant un alésage traversé par ladite tige, une fente débouchant axialement dans l'alésage, deux branches partagées par la fente, et des lumières oblongues ménagées dans les branches perpendiculairement à celles-ci et permettant un débattement d'un tronçon fileté d'un élément. La face externe de l'une des branches de la seconde attache peut être partiellement crantée. Le contour de la lumière dans l'une des branches de la seconde attache peut avoir au moins partiellement un profil transversal semi-circulaire.

Comme l'attache définie précédemment selon l'invention, la seconde attache peut être serrée contre une tête d'un élément par un écrou par l'intermédiaire d'un moyen pour amortir, et/ou par l'intermédiaire d'une rondelle à rotule sous-jacente à l'une des lumières de branche de l'attache, et/ou par l'intermédiaire d'un moyen traversé par le tronçon fileté de l'élément et disposé entre l'attache et l'écrou pour fournir une surface d'appui de l'écrou perpendiculaire au tronçon fileté.

D'autres caractéristiques et avantages de la présente invention apparaîtront plus clairement à la lecture de la description suivante de plusieurs réalisations préférées de l'invention en référence aux dessins annexés correspondants dans lesquels :
- la figure 1 est une vue schématique en perspective d'un dispositif de correction rachidienne conforme à l'invention, disposée avec des vis et des crochets pédiculaire et transversaire le long de deux courbures rachidiennes ;
- la figure 2 est une vue en perspective éclatée d'une vis pédiculaire autotaraudeuse avec une attache de liaison à deux branches pour pincer une tige de consolidation et d'autres pièces serrées avec l'attache ;
- la figure 3 est une vue en perspective éclatée de l'attache de la figure 2 avec des rondelles amortissante et une rondelle à rotule sous-jacentes à l'attache et une double rondelle de correction d'angulation sus-jacente à l'attache ;
- la figure 4 est une vue en coupe axiale de la vis et de l'attache montrées à la figure 2, l'attache étant serrée par l'intermédiaire desdites pièces sur la tête de la vis d'une manière inclinée par rapport à l'axe de la vis ;
- la figure 5 est une vue en perspective éclatée d'une attache additionnelle conforme à l'invention avec un crochet pédiculaire, une double rondelle crantée ou une rondelle plate crantée et un écrou ;
- les figures 6, 7 et 8 sont des vues de côté, de face et de dessus du crochet pédiculaire respectivement ;
- la figure 9 est une vue en perspective d'un crochet transversaire "incliné" vers la droite, conforme à l'invention ;
- les figures 10, 11 et 12 sont des vues de côté, de face et prise suivant la flèche F12 dans la figure 11 du crochet transversaire incliné vers la droite respectivement ; et
- la figure 13 est une vue en perspective d'un crochet transversaire "incliné" vers la gauche, conforme à l'invention.

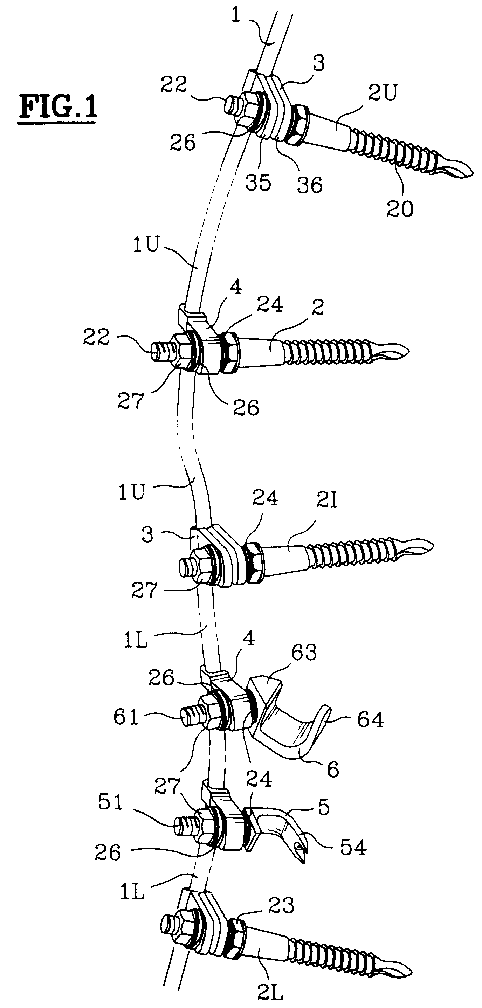

Un dispositif d'ostéosynthèse de correction rachidienne selon l'invention comprend des éléments vertébraux à implanter préalablement à l'introduction d'une tige de consolidation cintrée 1 qui peut présenter une seule courbure, ou bien deux courbures comme illustré à la figure 1. Par exemple, les courbures d'un tronçon supérieur 1U de la tige et d'un tronçon inférieur 1L de la tige sont adaptées respectivement à corriger une convexité cyphotique et une concavité lordosique dans un plan sagittal (antéro-postérieur), tout en étant adaptées à corriger des courbures scoliotiques dans un plan frontal.

Aux extrémités des courbures sont implantés des éléments vertébraux sous la forme de vis pédiculaires autotaraudeuses 2U, 2I, 2L, bien que certains de ces éléments puissent être des crochets pédiculaires ou transversaires, ou des paires de crochets pédiculaires ou transversaires comme exposé dans la suite. Ces éléments vertébraux peuvent être pour partie analogues à ceux décrits dans la demande de brevet EP-A-0 596 788, et pour partie conformes à l'invention.

Ces divers éléments et d'autres cités par la suite sont essentiellement métalliques, par exemple en un alliage à base de titane.

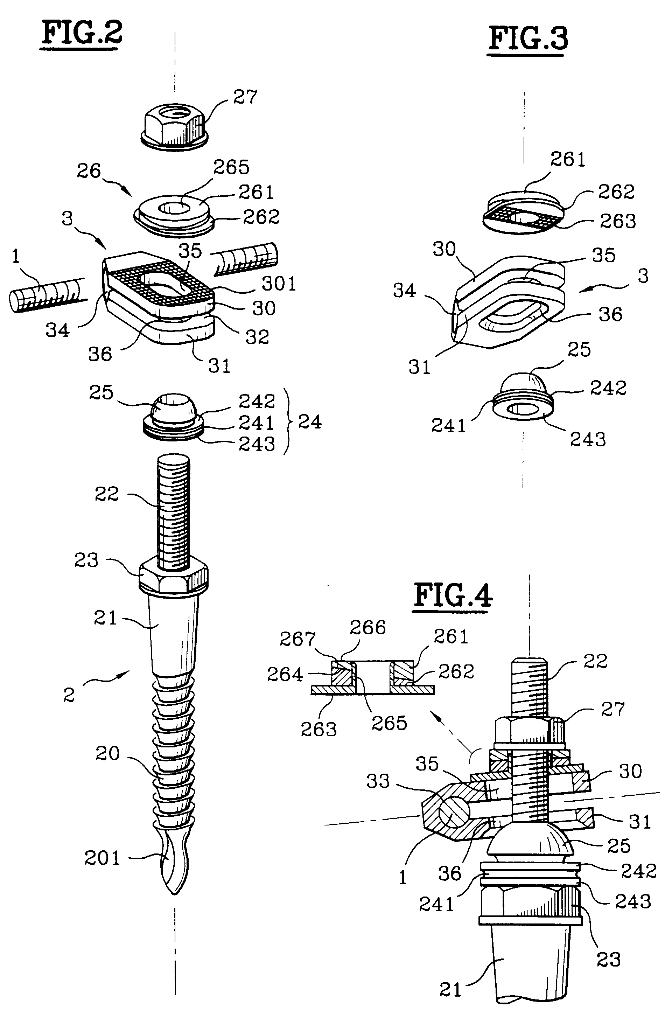

Les figures 2, 3 et 4 montrent une vis pédiculaire 2 avec une attache de liaison 3 pour relier la vis à la tige 1.

La vis pédiculaire 2 est composée d'une partie partiellement filetée 20-21 sensiblement tronconique, d'un tronçon cylindrique 22 fileté sensiblement sur toute sa longueur, et d'une tête de préhension intermédiaire 23, ici à section hexagonale, située entre les partie et tronçon filetés.

La partie 20-21 comprend, sensiblement sur la moitié de sa longueur, un tronçon sensiblement tronconique 20 ayant un pas de filetage rond relativement large, terminé par une extrémité autotaraudeuse à pointe mousse 201, c'est-à-dire une pointe comportant deux rainures axiales opposées avec une section en vé, de manière à pénétrer facilement et être ancré intimement dans l'os spongieux d'un corps vertébral. Un tronçon tronconique 21 disposé entre le tronçon 20 et la tête 23 complète la partie de vis 20-21. Le tronçon 21 est recouvert d'un revêtement rugueux en titane pur poreux afin que ses micro-anfractuosités soient comblées par croissance osseuse du pédicule vertébral où il est implanté. La tête de préhension 23 est destinée à être prise par une clé de manoeuvre correspondante pour visser le tronçon fileté 20 dans les pédicule et corps vertébraux jusqu'à ce que la tête 23 bute contre l'os cortical.

Le second tronçon fileté 22 constitue l'extrémité postérieure extra-vertébrale de la vis 2 après implantation, et est destiné à coopérer avec l'attache de liaison 3 et à recevoir une double rondelle amortissante 24 et une rondelle à rotule 25 disposées sous-jacente à l'attache 3, entre la tête de vis 23 et l'attache 3, et une double rondelle de correction d'angulation 26 et un écrou 24 disposés sus-jacents à l'attache 3, comme montré aux figures 2 et 4. Le tronçon fileté postérieur 22 est conçu initialement relativement long de manière à adapter sa longueur par sciage pré-opératoire au niveau de l'une de gorges prévues à cet effet. La face extrême du tronçon fileté 22 avant sciage peut comporter une fente pour recevoir un tournevis, ou bien une extrémité polygonale, pour coopérer avec une clé de manoeuvre.

L'attache 3 est sensiblement sous la forme d'un petit pavé partagé en des branches postérieure et antérieure 30 et 31 à implanter sensiblement "verticalement", par une fente 32 sensiblement dans un plan frontal. Les branches postérieure et antérieure de l'attache 3 naissent depuis une extrémité trapézoïdale dans laquelle un alésage lisse 33 recevant à coulissement la tige 1 est pratiqué. Des fraisures 34 aux extrémités de l'alésage 33 facilitent la pénétration de la tige 1 dans celui-ci. Les branches postérieure et antérieure de l'attache 3 sont percées perpendiculairement à l'alésage 3 pour former des lumières oblongues lisses 35 et 36, non concourantes avec l'alésage 33. Les lumières 35 et 36 ont une longueur suivant une direction perpendiculaire à l'alésage 33 supérieure aux diamètres externes des rondelles 24 à 26, mais une largeur inférieure à ces diamètres externes et sensiblement supérieure au diamètre du tronçon fileté 22 de la vis 2.

La double rondelle amortissante 24 constitue un moyen traversé par le tronçon fileté 22 de la vis 2 et disposé entre la tête de vis 23 et l'écrou 27 pour amortir des micro-déplacements relatifs entre la tige 1 et la vis 2. Ce moyen pour amortir annule des micro-déplacements rachidiens d'une vertèbre à l'autre et donc d'une vis pédiculaire à l'autre et ainsi évite des fêlures ou fractures de la vis 2 et/ou de la tige 1. La double rondelle 24 comprend une rondelle 241 en matériau amortissant, tel qu'élastomère ou polyuréthane ou silicone, enfilée sur le tronçon fileté 22. Selon la réalisation illustrée aux figures 2 à 4, la rondelle 241 est entretoisée par deux rondelles de guidage plates métalliques 242 et 243, qui sont de préférence thermocollées à la rondelle 241. La double rondelle amortissante 24 est enfilée autour du tronçon 22 et est enserrée à la base du tronçon 22, entre la tête de vis 23 et une base antérieure de la rondelle à rotule 25.

Selon d'autres variantes, ce premier assemblage de rondelles ne peut comporter qu'une rondelle 241 en matériau amortissant et une seule rondelle métallique 242 ou 243.

La rondelle à rotule 25 comporte une jupe qui est solidaire de la rondelle plate postérieure sous-jacente 242 et qui s'enfile à glissement autour de la tige filetée 22. La jupe de la rondelle 25 peut être emmanchée à force dans la rondelle 242, ou être monolithique avec la rondelle 242. La rotule de la rondelle 25 est une calotte semi-sphérique dont la hauteur est sensiblement égale à l'épaisseur de la branche antérieure 31 de l'attache 3 et qui est complémentaire du profil transversal sensiblement semi-circulaire du contour de la lumière 36 dans la branche 31 (figure 3). La longueur de la lumière 36 est supérieure au diamètre de l'extrémité semi-sphérique de la rondelle à rotule et la largeur de la lumière 36 est inférieure au diamètre de l'extrémité semi-sphérique de la rondelle à rotule. L'attache 3 peut ainsi reposer sur la rotule suivant un plan incliné par rapport à l'axe de la vis 2, avec des angles maximums de l'ordre de 12° suivant la longueur des lumières 35 et 36 et de l'ordre de 5° suivant la largeur des lumières.

La double rondelle de correction d'angulation 26 comprend une rondelle postérieure en coin 261 et une rondelle antérieure en coin 262 qui sont solidaires avec jeu l'une avec l'autre en direction axiale et peuvent tourner librement l'une par rapport à l'autre.

La rondelle 262 a une face antérieure crantée 263, comme la face crantée postérieure 301 de la branche d'attache postérieure 30, et une face postérieure lisse 264 inclinée de 3° environ par rapport à la face antérieure 263, c'est-à-dire par rapport à un plan perpendiculaire à l'axe de la double rondelle 26. Une jupe postérieure mince 265 de la rondelle 262 saille de la face postérieure 264 et est entourée par l'autre rondelle de correction angulaire 261. Une collerette antérieure de la rondelle 261 est surmontée par une collerette postérieure en bout de la jupe 265 de manière à retenir avec jeu la rondelle 261 contre la rondelle 262, tout en offrant la possibilité de tourner la rondelle 261 sur la rondelle 262.

La rondelle 261 également en forme de coin a une face postérieure 266 perpendiculaire à l'axe de la rondelle et une face antérieure 267 inclinée par rapport à la face postérieure de 3° environ.

Grâce à la rotation de la face inclinée 267 de la rondelle postérieure 262 sur la face inclinée 264 de la rondelle antérieure 262, l'angle entre la face postérieure 266 de la rondelle 261 contre laquelle l'écrou 27 s'appuie et la face antérieure crantée 263 de la rondelle 262 peut varier de O° lorsque les faces 266 et 263 sont parallèles et les portions épaisses des rondelles 261 et 262 sont diamétralement opposées, jusqu'à 6° lorsque les faces 266 et 263 sont inclinées au maximum l'une par rapport à l'autre et les portions épaisses des rondelles 261 et 262 sont superposées, comme montré à la figure 4.

La double rondelle de correction d'angulation 26 compense ainsi le défaut de perpendicularité des branches d'attache 30 et 31 par rapport à l'axe de la vis 2, qui est possible grâce au débattement du tronçon fileté 22 dans les lumières oblongues 35 et 36, afin d'assurer un serrage efficace de l'attache 3 en vissant l'écrou 27 contre une face d'appui formée par la face postérieure 266 de la rondelle 261 parfaitement perpendiculaire à l'axe du tronçon fileté 22 et en appliquant la face antérieure 263 de l'autre rondelle 262 parfaitement contre la branche d'attache 30, pour ne pas fausser les filetages de l'écrou et du tronçon fileté 22, et afin de pousser l'attache 3 contre la tête de vis 23 en répartissant la poussée grâce à la rondelle à rotule 25 pour que l'attache 3 pince la tige 1. Ainsi, l'attache 3 n'a pas besoin d'être perpendiculaire à la vis 2 pour relier solidement la vis à la tige de consolidation 1. Ceci offre toute lattitude pour positionner des vis 2 le long de la tige 1 avec des axes de vis inclinés indifféremment les uns par rapport aux autres.

Conformément à l'invention, au moins une attache additionnelle 4 est introduite sur la tige de consolidation 1 entre deux vis 2 déjà implantées, de manière à relier la tige 1 à une vis 2 ou un crochet à implanter, comme montré à la figure 1. Par exemple en fonction de la mobilité souhaitée de l'une des vertèbres lombaires entre la vis supérieure 2U et la vis intermédiaire 2I dans la figure 1, une attache 4 est ajoutée sur la tige 1 au niveau d'une vertèbre lombaire prédéterminée de manière à défléchir la tige 1 en tirant la tige vers la droite, ce qui détracte la concavité de la courbure rachidienne lorsque la tige est positionnée du côté concave de celle-ci, ou ce qui compresse la convexité de la courbure rachidienne lorsque la tige est positionnée du côté convexe de celle-ci.

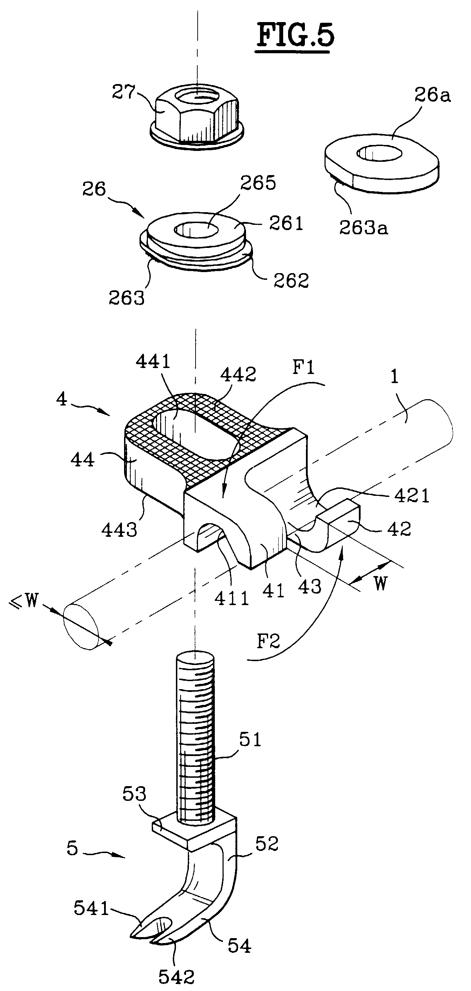

Comme montré à la figure 5, une attache additionnelle 4 comprend, comme une attache de liaison 3, une première portion pour la relier à la tige 1 et une seconde portion pour la relier à la tige filetée d'une vis 1 ou d'un crochet.

La première portion comprend deux branches parallèles 41 et 42 qui sont conformées en crochet et qui sont disposées en regard, mais décalées le long de la tige. Les crochets 41 et 42 offrent des surfaces d'appui concaves 411 et 421, coaxiales, sensiblement semi-cylindriques et évasées à l'opposé de la seconde portion, ayant un rayon sensiblement égal à celui de la tige de consolidation lorsque celle-ci est cylindrique. Si la section de la tige est polygonale, par exemple carrée ou hexagonale, les surfaces d'appui 411 et 421 épousent sensiblement des demi-sections transversales opposées de la tige. L'espace 43 entre les crochets 41 et 42 le long de l'axe de ceux-ci a une largeur W au moins égale au diamètre de la tige 1, ou plus généralement au moins égale à la plus petite dimension de la section transversale de la tige. Dans ces conditions, l'attache additionnelle 4 est présentée avec un axe des surfaces d'appui de crochet 411 et 421 perpendiculaire à la tige immobile 1, afin que l'espace 43 entre les crochets d'attache contienne un tronçon de la tige 1. Puis l'attache 4 est tournée de 90° suivant les flèches F1 et F2 autour d'un axe de rotation perpendiculaire à la tige 1 et à l'axe des surfaces d'appui 411 et 421 jusqu'à ce que celles-ci butent, comme un bras de levier, contre la tige 1 et soient diamétralement opposées et décalées sur la tige 1.

La seconde portion de l'attache additionnelle 4 comprend une queue parallélépipédique plate 44 analogue à la superposition des branches 30 et 31 de l'attache de liaison 3. La queue 44 est sensiblement plus épaisse que chaque branche 30, 31, et a une épaisseur sensiblement égale à chaque crochet 41, 42. Une lumière oblongue 441 de dimensions analogues à celles des lumières 35 et 36 traverse la queue plate 44 perpendiculairement à ses faces 442 et 443, et également perpendiculairement à l'axe des surfaces d'appui de crochet 411 et 421. La face postérieure 442 de la queue 44 est crantée, comme la face postérieure d'attache de liaison 301, de manière à coopérer avec la face antérieure crantée 263 d'une double rondelle de correction d'angulation 26. Comme montré à la figure 5, la double rondelle 26 peut être remplacée par une rondelle plate 26a avec une face antérieure crantée 263a.

Selon une variante, du côté de la face antérieure 443, le profil transversal du contour de la lumière 441 est sensiblement semi-circulaire, comme celui de la lumière 36 de l'attache de liaison 3 (figure 3), afin qu'une rotule semi-sphérique de rondelle 25 glisse contre ce contour et adapte l'orientation de l'axe du tronçon fileté 22 d'une vis 2, ou celui d'un crochet, à ajouter et à solidariser à la tige 1, à l'inclinaison relative de l'attache 4.

Selon la réalisation illustrée à la figure 5, la queue 44 de l'attache additionnelle 4 a une épaisseur répartie également de part et d'autre d'un plan de "quasi-symétrie" de l'attache contenant l'axe des surfaces d'appui 411 et 421. Selon une autre variante, la face antérieure 443 est sensiblement coplanaire à la face frontale plane de l'un 42 des crochets d'attache, et la face postérieure 442 est dans un plan contenant l'axe des surfaces d'appui 411 et 421, afin de serrer l'attache 4 à la vis 2 ou au crochet pédiculaire ou transversaire le plus près possible du corps de la vertèbre prédéterminée.

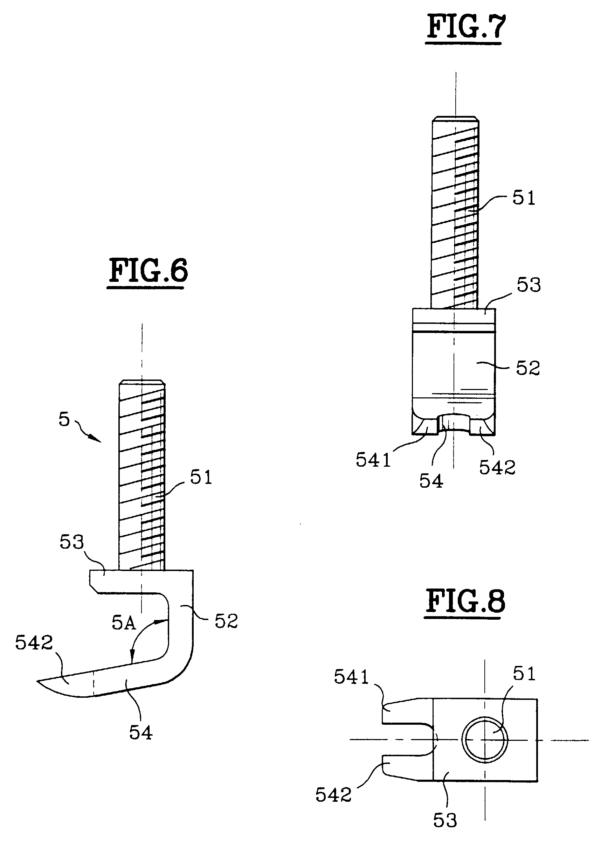

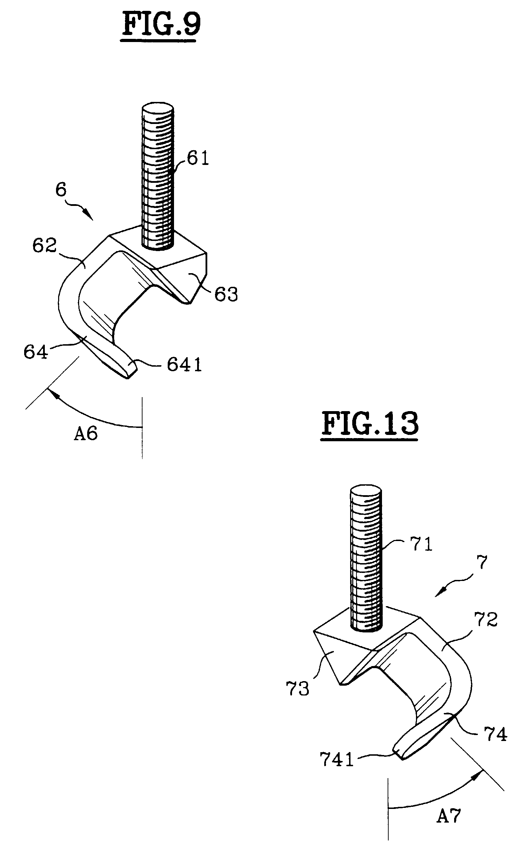

Comme déjà dit, à la place d'une vis pédiculaire 2, un crochet peut être relié à la tige de consolidation 1. Le crochet peut être
soit analogue à un crochet dit "première griffe vertébrale" montrée à la figure 4 de la demande de brevet EP-A-0 596 788, ayant une partie en crochet plus ou moins mince avec une extrémité effilée sensiblement perpendiculaire au tronçon fileté,
soit un second crochet fendu 5 comme montré aux figures 5 à 8,
ou un troisième crochet 6 "incliné" vers la droite comme montré aux figures 9 à 12, ou 7 "incliné" vers la gauche comme montré à la figure 13.

En référence aux figures 5 à 8, le crochet pédiculaire 5 comporte également un tronçon fileté cylindrique 51 propre à traverser les lumières 35 et 36 d'une attache 3, ou la lumière 441 d'une attache 4 afin de lier le crochet 5 à l'attache par des pièces 24 à 27 ou 26 et 27, comme pour la vis 2 montrée à la figure 4. A titre d'exemple, la figure 1 montre un assemblage de crochet 5 avec une attache additionnelle 4 disposé entre les vis 21 et 2U.

Sous-jacente au tronçon fileté 51 du crochet 5 est prévue une partie en forme de dièdre 52 à arête arrondie et angle plan 5A sensiblement droit compris entre 90° et 100°, formant un "crochet" ou une "griffe". L'extrémité d'un côté de la partie en crochet 52 est sous-jacente à un côté d'une tête intermédiaire rectangulaire plate 53 formant une base perpendiculaire au tronçon fileté 51. L'autre côté libre de la partie en crochet 52 est constitué par une lame 54 inclinée vers le bas d'au plus 10° par rapport à la tête 53 et plus longue que la tête 53 de manière à faciliter une prise pédiculaire par exemple sur des vertèbres lombaires. La lame 54 peut être terminée en forme de fourche à deux dents 541 et 542 pour enfourcher un pédicule. La lame de crochet 54 est destinée à accrocher des bordures de divers évidements des vertèbres notamment pédiculaires, sur des vertèbres lombaires et thoraciques. La partie en forme de dièdre 52 peut être orientée selon une direction quelconque grâce à la rotation du tronçon fileté 51 dans les lumières 35 et 36 ou la lumière 441.

Chacun des troisièmes crochets 6 et 7, dits crochets transversaires, montrés aux figures 9 et 13 comporte également un tronçon fileté 61, 71 propre à traverser les lumières 35 et 36 d'une attache 3 ou la lumière 41 d'une attache 4 afin de lier le crochet 6, 7 à l'attache par des pièces 24 à 27, comme pour la vis 2 montrée à la figure 4.

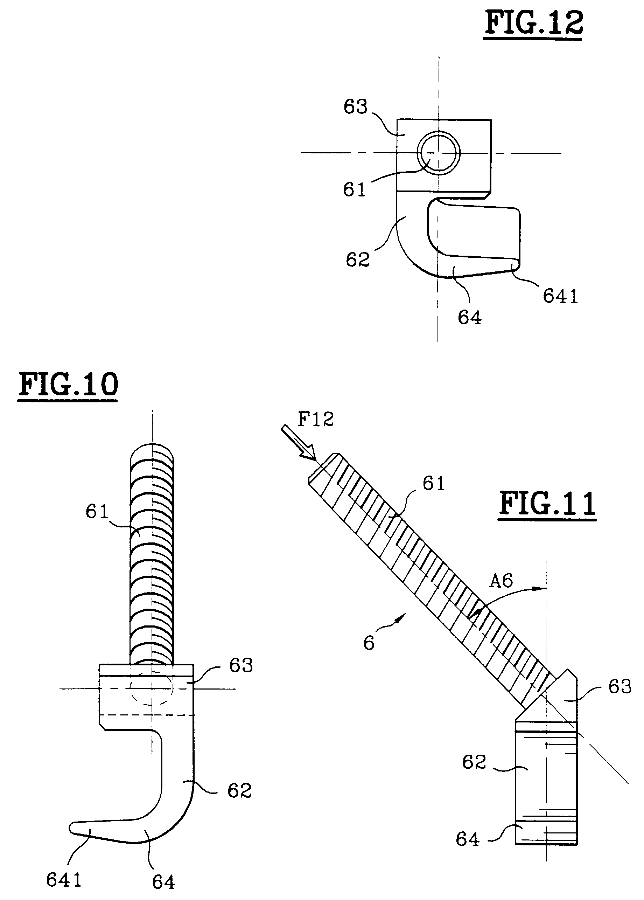

Les crochets 6 et 7 sont symétriques l'un de l'autre par rapport aux axes des tronçons filetés 61 et 71. Seulement l'un d'eux, le crochet 6 "incliné" vers la droite est montré en détail aux figures 10, 11 et 12.

Le crochet 6, 7 comporte une partie en forme de dièdre sensiblement droit 62, 72 qui est "penchée" vers la droite ou vers la gauche par rapport à l'axe du tronçon fileté 61, 71. Ainsi, une tête intermédiaire 63, 73 du crochet 6, 7 entre la partie en dièdre 62, 72 et le tronçon fileté 61, 71 a un profil triangulaire parallèlement à l'axe du tronçon fileté. Le côté de la partie en dièdre 62, 72 lié à une arête de la tête 63, 73 s'étend suivant un angle A6, A7 de l'ordre de 40° à 50°, typiquement 45°, par rapport à l'axe du tronçon fileté 61, 71. L'autre côté de la partie en dièdre 62, 72 comporte une lame plate 64, 74 dont la section transversale diminue vers un arrondi d'extrémité 641, 741 comme montré aux figures 10 ou 12, ou par une fourche à deux dents d'une manière analogue à la lame de crochet 54 montrée à la figure 5. L'angle plan du dièdre de la partie 62, 72 est compris entre 90° et 100°.

Les crochets transversaires 6 et 7 "inclinés" vers la droite et vers la gauche servent à atteindre notamment les transverses thoraciques dont les faces d'appui sont inclinées par rapport à un plan frontal dans lequel la tige de consolidation 1 est au moins partiellement développée.

## Revendications

1. Dispositif de correction rachidienne comprenant une tige (1), un élément (2, 5, 6, 7) ayant un tronçon fileté (22, 51, 61, 71) s'étendant à partir d'une tête (23, 53, 63, 73) et muni d'un écrou (27), et une attache ayant un orifice traversé par le tronçon fileté pour lier ledit élément à la tige, caractérisé en ce que l'attache (4) comprend deux surfaces d'appui (411, 421) qui sont opposées sensiblement transversalement à la tige (1) et décalées le long de la tige d'au moins la plus petite dimension (W) de la section transversale de la tige (1).

2. Dispositif conforme à la revendication 1, dans lequel les deux surfaces d'appui (411, 421) ont des sections transversales similaires à des demi-sections transversales opposées de la tige (1).

3. Dispositif conforme à la revendication 1 ou 2, dans lequel les surfaces d'appui (411, 421) sont celles de deux crochets (41, 42) saillant d'une queue (44) de l'attache (4) dans laquelle l'orifice (441) traversé par le tronçon fileté (22, 51, 61, 71) est ménagé.

4. Dispositif conforme à l'une quelconque des revendications 1 à 3, comprenant un moyen (24) traversé par le tronçon fileté (22, 51, 61, 71) et disposé entre la tête d'élément (23, 53, 63, 73) et l'écrou (27) pour amortir des déplacements relatifs entre la tige (1) et ledit élément (2, 5, 6, 7).

5. Dispositif conforme à la revendication 4, dans lequel le moyen pour amortir comprend une rondelle en matériau amortissant (241) qui est de préférence en contact avec ou liée à l'une ou deux rondelles métalliques (242, 243).

6. Dispositif conforme à l'une quelconque des revendications 1 à 5, comprenant une rondelle à rotule (25) traversée par le tronçon fileté (22, 51, 61, 71), disposée sous-jacente à l'orifice (441) de l'attache (4), entre celle-ci et la tête d'élément (23, 53, 63, 73), ledit orifice (441) étant une lumière oblongue ayant au moins partiellement un profil transversal de contour semi-circulaire (444) coopérant avec une extrémité semi-sphérique de la rondelle à rotule (44).

7. Dispositif conforme à la revendication 6, dans lequel la longueur de la lumière (36) est supérieure au diamètre de l'extrémité semi-sphérique de la rondelle à rotule (25) et la largeur de la lumière (36) est inférieure au diamètre de l'extrémité semi-sphérique de la rondelle à rotule (44).

8. Dispositif conforme à la revendication 5 et à la revendication 6 ou 7, dans lequel la rondelle à rotule (25) et une rondelle métallique (242) du moyen pour amortir sont monolithiques.

9. Dispositif conforme à l'une quelconque des revendications 1 à 8, comprenant un moyen (26) traversé par le tronçon fileté (22, 51, 61, 71) et disposé entre ladite attache (4) et l'écrou (27) pour fournir une surface d'appui de l'écrou (27) perpendiculaire au tronçon fileté, indépendamment de l'angle entre le tronçon fileté (22, 51, 61, 71) et l'attache (4).

10. Dispositif conforme à la revendication 9, dans lequel le moyen (26) pour fournir une surface d'appui de l'écrou présente une surface crantée (263) pour être appliquée contre une face crantée (442) de l'attache (4).

11. Dispositif conforme à la revendication 9 ou 10, dans lequel le moyen pour fournir une surface d'appui de l'écrou comprend deux rondelles en coin (261, 262).

12. Dispositif conforme à la revendication 11, dans lequel les deux rondelles en coin sont solidaires avec jeu en direction axiale et sont libres en rotation axiale l'une par rapport à l'autre.

13. Dispositif conforme à l'une quelconque des revendications 1 à 12, dans lequel ledit élément (2) comporte une partie (21) au moins partiellement filetée et séparée dudit tronçon fileté (22) par la tête (23).

14. Dispositif conforme à l'une quelconque des revendications 1 à 12, dans lequel ledit élément (5) comporte une partie de crochet (52) en forme de dièdre sensiblement droit séparée dudit tronçon fileté (51) par la tête (53).

15. Dispositif conforme à la revendication 14, dans lequel ladite partie de crochet en forme de dièdre comporte un côté libre (54) en forme de lame terminée par deux dents (541, 542).

16. Dispositif conforme à l'une quelconque des revendications 1 à 12, dans lequel ledit élément (6, 7) comporte une partie de crochet (62, 72) en forme de dièdre sensiblement droit dont l'angle plan (A6, A7) est incliné par rapport au tronçon fileté (61, 71) et qui est séparée dudit tronçon fileté (61, 71) par la tête (63, 73).

17. Dispositif conforme à l'une quelconque des revendications 1 à 16, comprenant une seconde attache (3) ayant un alésage (33) traversé par ladite tige (1), une fente (32) débouchant axialement dans l'alésage et deux branches (30, 31) partagées par ladite fente, caractérisé par des lumières oblongues (35, 36) ménagées dans les branches perpendiculairement à celles-ci et audit alésage permettant un débattement d'un tronçon fileté (22, 51, 71) d'un élément (2, 5, 6, 7).

18. Dispositif conforme à la revendication 17, dans lequel la face externe (301) de l'une (30) des branches de la seconde attache (3) est partiellement crantée.

19. Dispositif conforme à la revendications 17 ou 18, dans lequel le contour de la lumière (36) dans l'une (31) des branches de la seconde attache (3) a au moins partiellement un profil transversal semi-circulaire.

20. Dispositif conforme à l'une quelconque des revendications 17 à 19, dans lequel la seconde attache est serrée contre une tête (23, 53, 63, 73) d'un élément (2, 5, 6, 7) par un écrou (27) par l'intermédiaire d'un moyen pour amortir (24) conforme à la revendication 4 ou 5, et/ou par l'intermédiaire d'une rondelle à rotule (25) conforme à l'une quelconque des revendications 6 à 8 et sous-jacente à l'une (31) des lumières de branches de l'attache (3), et/ou par l'intermédiaire d'un moyen (26) conforme à l'une des revendications 9 à 12 et traversé par le tronçon fileté (22, 51, 61, 71) dudit élément et disposé entre ladite attache (3) et l'écrou (27) pour fournir une surface d'appui de l'écrou (27) perpendiculaire au tronçon fileté.
